Europäisches Patentamt

European Patent Office

Office européen des brevets

(19)

(11) Publication number: **0 041 751**

**B1**

(12) # EUROPEAN PATENT SPECIFICATION

(45) Date of publication of patent specification: 24.10.84    (51) Int. Cl.³: **C 07 D 201/16**

(21) Application number: **81200578.3**

(22) Date of filing: **29.05.81**

(54) Process for the purification of 2-pyrrolidone.

(30) Priority: **10.06.80 NL 8003365**

(43) Date of publication of application:
**16.12.81 Bulletin 81/50**

(45) Publication of the grant of the patent:
**24.10.84 Bulletin 84/43**

(84) Designated Contracting States:
**BE DE FR GB IT NL**

(56) References cited:
**EP-A-0 022 161**
**DE-A-2 209 257**
**DE-A-2 550 934**
**GB-A- 846 575**
**US-A-2 964 535**
**US-A-4 050 994**

**CHEMICAL ABSTRACTS, vol. 76, no. 1, January 3, 1972, page 304, abstract 3417w, COLUMBUS, OHIO (US)**

(73) Proprietor: **STAMICARBON B.V.**
**Postbus 10**
**NL-6160 MC Geleen (NL)**

(72) Inventor: **Goettsch, Reijer**
**Aldenhofstraat 15**
**NL-6191 GP Beek (L.) (NL)**
Inventor: **Jetten, Arnold Godfried Maria**
**Rector Thijssenstraat 1**
**NL-6237 NG Ulestraten (NL)**

(74) Representative: **Hoogstraten, Willem Cornelis Roeland et al**
**OCTROOIBUREAU DSM Postbus 9**
**NL-6160 MA Geleen (NL)**

Courier Press, Leamington Spa, England.

## Description

The invention relates to a process for the purification of 2-pyrrolidone to render this suitable for polymerization to polypyrrolidone having a sufficiently high molecular weight.

A suitable method for the preparation of 2-pyrrolidone is the hydrogenation of succinonitrile followed by reaction with water of the hydrogenation product formed (see for instance the United States Patents, 3,644,402, 4,123,438 and 4,193,925). When using this method an aqueous pyrrolidone-containing solution is obtained from which, for instance by fractional distillation, pyrrolidone having a good purity can be recovered. When the pyrrolidone thus obtained is used for the preparation of polypyrrolidone, however, the result of the polymerization is not very satisfactory.

Many methods have already been proposed for the purification of pyrrolidone, which, however, are not very suitable because, for instance, too much pyrrolidone is lost, or the method in question can be applied only to pyrrolidone obtained in another way than from succinonitrile, or because the degree of purity reached does not sufficiently agree with the degree of purity reached does not sufficiently agree with the degree of purity required for polymerization of the pyrrolidone.

According to a fairly recent method (see United States Patent 4,014,900) the pyrrolidone can be purified by contacting it in liquid condition with a water-free hydroxide such as for instance KOH and subsequently subjecting the resulting product to a molecular distillation. This method yields a good degree of purity, but the pyrrolidone losses amount to 5—10%.

It is known from Chemical Abstracts 76: 1317 W to dissolve the residue obtained by distilling crude ε-caprolactam in the presence of alkali metal hydroxides in aqueous ethanol or methanol, neutralize, filter and distil in vacuo in the presence of phosphoric acid. In this process it is not possible to recover potassium hydroxyde from the residue.

The present invention provides a method for the purification of pyrrolidone obtained from succinonitrile which allows a good product to be obtained while keeping the pyrrolidone losses low.

The process according to the invention for the purification of 2-pyrrolidone obtained from succinonitrile, whereby the product to be purified is contacted in liquid form with a strong base and subjected to a distillation, is characterized in that the pyrrolidone to be purified is treated in liquid form with potassium or sodium hydroxyde at a temperature between 80 and 150°C, thereby removing any water, the product obtained is subjected to a fractional distillation yielding purified pyrrolidone, and the pyrrolidone-containing residue of this distillation is worked up by mixing it with water and carbonic acid and subjecting the mixture obtained to an extraction.

In the process according to the invention, working up of the residue yields pyrrolidone which as such is suitable for conversion into various other products, for instance N-vinyl pyrrolidone or N-methyl pyrrolidone. This pyrrolidone can, however, also be recirculated to the aqueous reaction mixture yet to be worked up that is obtained in the preparation of pyrrolidone from succinonitrile, so that ultimately only pyrrolidone having the grade of purity required for polymerization is obtained.

The treatment of the pyrrolidone in liquid condition with potassium or sodium hydroxide is carried out by preference at a temperature between 110 and 130°C. The duration of this treatment may vary, for instance between 10 minutes and 5 hours. A treatment having a duration in excess of 5 hours can also be applied but does not yield any advantage. A very satisfactory result can be obtained with a treatment duration of 0.5—3 hours. For this treatment preferably potassium hydroxide is applied. The amount of potassium or sodium hydroxide required is only slight, for instance 0.2—2 g per 100 g pyrrolidone to be purified. The most suitable amount of the hydroxide is partly determined by the amount of impurities. Usually an amount of the hydroxide of 0.5—1.5 g per 100 g pyrrolidone to be purified gives very satisfactory results.

In principle the fractional distillation of the product obtained after treating the pyrrolidone with the hydroxide can be carried out at atmospheric pressure. By preference this distillation is carried out at reduced pressure, for instance a pressure of 0.1—0.3 bar.

The residue of the fractional distillation is according to the invention mixed with water, for instance 20—70 g water per 100 g residue, and carbonic acid. Then a potassium or sodium carbonate is formed from the hydroxide present in the residue as a compound with pyrrolidone, and from this carbonate the corresponding hydroxide can be recovered in pure form. The amount of carbonic acid is chosen so that the corresponding hydroxide in the residue can in its entirety be converted into carbonate.

The extraction of the residue-containing aqueous mixture can very suitably be carried out countercurrently using solvents such as, for instance, benzene, toluene and xylenes at temperatures of, for instance, 20—50°C. Per gram of pyrrolidone to be extracted an amount of for instance 1—9 g extraction agent is required.

The recovery of the pyrrolidone from the organic phase obtained in the extraction can for instance be effected by evaporation of the solvent or by having the pyrrolidone crystallize. If desired, the organic phase can be subjected to an extraction with water. In that case an aqueous pyrrolidone-containing solution which can be recirculated to the aqueous reaction mixture obtained in the preparation of pyrrolidone from succinonitrile.

In the following example the invention will be further elucidated.

Example

In the distillation flask of a vacuum distillation apparatus 1050 g pyrrolidone (99% purity, obtained from succinonitrile in the way described in United States Patent 4,123,438) is mixed with 8.76 g KOH at a temperature of approximately 45°C. The mixture obtained is then given a temperature of 120°C in a nitrogen atmosphere. In doing so, the pressure is reduced so far below 1 bar that the slight amount of water, present in the potassium hydroxide and the impure pyrrolidone, as well as 50 g pyrrolidone distill over. Subsequently, the mixture is for one hour maintained at 120°C in the distillation flask at a higher pressure (below 1 bar) without being allowed to boil. Then the pressure is again reduced to a value at which the mixture boils and the pyrrolidone distills over.

Thus 946.8 g purified pyrrolidone is obtained. This pyrrolidone is colourless and does not discolour when stored in a brown bottle in a nitrogen atmosphere for one month.

The distillation residue (59.1 g) is dissolved in 16.2 g water and mixed with 60 g toluene. Subsequently, carbon dioxide is passed through the mixture, which is meanwhile being stirred, until the pH is approximately 13.2. The potassium pyrrolidonate present in the residue has then been converted into pyrrolidone and potassium carbonate. The supernatant toluene layer is then separated off and the remaining mixture is extracted four times, each time with 60 g toluene. After evaporation of the toluene from the toluene layers that have been combined, 54 g pyrrolidone (of 98.8% purity) is obtained that is suitable for conversion into, for instance, N-vinyl pyrrolidone, or that can be recirculated to the section where the reaction mixture obtained in the preparation of pyrrolidone from succinonitrile is worked up.

In the extraction with toluene a contaminated potassium carbonate-water mixture remains behind from which potassium hydroxide can be recovered in a known way.

Polymerization and Determination of the Colour of the Purified Pyrrolidone

In a nitrogen atmosphere 120 ml of the purified pyrrolidone obtained is introduced into 250 ml flask provided with a distillation column, after which the pyrrolidone is heated to the boiling point under vacuum conditions. The temperature in the top of the distillation column then is about 96°C and that in the flask about 125°C. Subsequently, solid potassium hydroxide (0.1 mole KOH per mole pyrrolidone in 100 ml pyrrolidone) is added in a nitrogen atmoshere, and the reaction water formed is distilled off under vacuum conditions within a minute. In addition, 20 ml pyrrolidone is distilled off. The remaining residue is rapidly cooled to 40°C in a nitrogen atmosphere.

Part of the residue is cooled further to 25°C and subsequently the extinction at 390 nm of this part is measured in a glass cell. The colour index number is 9° Hazen (if, for comparison purposes, the purification according to the example given is carried out without keeping the mixture at 120°C for one hour before the distillation, all other conditions remaining unchanged, the colour index number is 100° Hazen).

To the remaining part of the residue, carbon dioxide is added in an amount of 0.3 mole per mole of potassium pyrrolidonate present, which is done at 40°C under vacuum in about 20 minutes' time, after which the mixture obtained is kept at a temperature of approximately 50°C for 24 hours in a nitrogen atmosphere. The white polymer obtained is ground, washed and dried. The polymerization conversion amounts to 50%. The relative viscosity (1 g polymer in 100 g $H_2SO_4$) is 46, corresponding with a molar weight of about 410,000.

Claims

1. Process for the purification of 2-pyrrolidone obtained from succinonitrile, whereby the product to be purified is contacted in liquid form with a strong base and subjected to a distillation, characterized in that the pyrrolidone to be purified is treated in liquid form with potassium or sodium hydroxide at a temperature between 80 and 150°C, thereby removing any water, the product obtained is subjected to a fractional distillation yielding purified pyrrolidone, and the pyrrolidone containing residue of this distillation is worked up by mixing it with water and carbonic acid and subjecting the mixture obtained to an extraction.

2. Process according to claim 1, characterized in that the treatment with a strong base is carried out at a temperature pf 110—130°C.

3. Process according to either of claims 1 and 2, characterized in that the treatment with a strong base is carried out in 0.5—3 hours.

4. Process according to any one of the claims 1—3, characterized in that potassium hydroxide is used for the said treatment.

5. Process according to any one of the claims 1—3, characterized in that 0.5—1.5 g potassium or sodium hydroxide is used per 100 g pyrrolidone to be purified.

6. Process according to any one of the claims 1—5, characterized in that the fractional distillation is carried out at a pressure of 0.1—0.3 bar.

Revendications

1. Procédé de purification de 2-pyrrolidone obtenue à partir de succinonitrile, par lequel on met en contact le produit à purifier sous forme liquide avec une base forte et on le soumet à une distillation, caractérisé en ce qu'on traite la pyrrolidone à purifier sous forme liquide avec de l'hydroxyde potassium ou de sodium à une température comprise entre 80 et 150°C, enlevant ainsi toute l'eau qui s'y trouve éventuellement, en ce qu'on soumet le produit obtenu à une distillation fractionnée donnant de la pyrrolidone purifiée, et en ce qu'on traite le résidu contenant de la pyrrolidone de cette distillation en le mélangeant avec de l'eau et de l'acide carbonique et en soumettant le mélange obtenu à une extraction.

2. Procédé selon la revendication 1, caractérisé en ce que le traitement avec une base forte est conduit à une température de 110—130°C.

3. Procédé selon l'une quelconque des revendications 1 et 2, caractérisé en ce que le traitement avec une base forte est conduit en 1/2 heure à 3 heures.

4. Procédé selon l'une quelconque des revendications 1—3, caractérisé en ce qu'on utilise de l'hydroxyde de potassium pour ledit traitement.

5. Procédé selon l'une quelconque des revendications 1—3, caractérisé en ce qu'on utilise de 0,5 à 1,5 g d'hydroxyde de potassium ou de sodium pour 100 g de pyrrolidone à purifier.

6. Procédé selon l'une quelconque des revendications 1—5, caractérisé en ce que la distillation fractionnée est conduite à une pression de 0,1 à 0,3 bar.

**Patentansprüche**

1. Verfahren zur Reinigung von 2-Pyrrolidon, gewonnen aus Bernsteinsäurenitril, wobei das zu reinigende Produkt in flüssiger Form mit einer starken Base in Kontakt gebracht und einer Destillation unterzogen wird, dadurch gekennzeichnet, daß das zu reinigende Pyrrolidon in flüssiger Form mit Kalium oder Natriumhydroxyd bei einer Temperatur von 80 bis 150°C behandelt wird, wobei alles Wasser entfernt wird und das gewonnene Produkt einer Teildestillation unterzogen wird, welche gereinigtes Pyrrolidon ergibt und wobei der Pyrrolidon enthaltende Rest dieser Destillation durch Vermischen mit Wasser und Kohlensäure aufgearbeitet und die erhaltene Mischung einer Extraktion unterzogen wird.

2. Verfahren nach Anspruch 1, dadurch gekennzeichnet, daß die Behandlung mit einer starken Base bei einer Temperatur von 110—130°C durchgeführt wird.

3. Verfahren nach einem der Ansprüche 1 und 2, dadurch gekennzeichnet, daß die Behandlung mit einer starken Base in 0,5—3 Stunden durchgeführt wird.

4. Verfahren nach einem der Ansprüche 1—3, dadurch gekennzeichnet, daß Kaliumhydroxyd für die besagte Behandlung verwendet wird.

5. Verfahren nach einem der Ansprüche 1—3, dadurch gekennzeichnet, daß 0,5—1,5 g Kalium oder Natriumhydroxyd je 100 g zu reinigendem Pyrrolidon verwendet wird.

6. Verfahren nach einem der Ansprüche 1—5, dadurch gekennzeichnet, daß die Teildestillation bei einem Druck von 0,1—0,3 bar durchgeführt wird.